# EUROPEAN PATENT APPLICATION

(11) **EP 3 275 495 A1**
(43) Date of publication of application: **31.01.2018**
(21) Application number: 15886487.6
(22) Date of filing: 12.11.2015
(51) Int. Cl.: A61M 25/01, A61B 1/00, A61B 17/32

(54) **TREATMENT INSTRUMENT**

(30) Priority: 25.03.2015 JP 2015062614
(71) Applicant: Olympus Corporation, Hachioji-shi Tokyo 192-8507 (JP)
(72) Inventor: MATSUNO, Kiyotaka, Kuroishi-shi Aomori 036-0357 (JP); NAKAMURA, Yoshisane, Tokyo 192-8507 (JP)
(74) Representative: Schicker, Silvia
(86) International application number: PCT/JP2015/081809
(87) International publication number: WO 2016/151935

(57) **Abstract**

A treatment tool includes a sheath that has a first opening portion which has a longitudinal axis, is formed with a lumen through which a guide wire is capable of being inserted along the longitudinal axis, and is opened to communicate on a distal end side of the lumen, and a second opening portion that is opened to an outer circumferential surface to communicate on a proximal end side of the lumen; a proximal end holding portion that holds a proximal end region in the proximal end portion of the sheath; and a distal end holding portion that bends and holds a distal end region of the proximal end portion of the sheath, and is disposed by being deflected to the proximal end holding portion while being continued to a distal end of the proximal end holding portion at an angle in which the second opening portion is located on the extension of the central axis of the lumen in the distal end region,

## Description

### [Technical Field]

The present invention relates to a treatment tool.

Priority is claimed on Japanese Patent Application No. 2015-062614, filed on March 25, 2015, the content of which is incorporated herein by reference.

### [Background Art]

As a technique for cutting a sphincter of a duodenal papilla, while observing the duodenal papilla using an endoscope apparatus, endoscopic sphincterotomy (hereinafter, referred to as EST) is known. In EST, for example, a guide wire is inserted into a common bile duct, and a treatment tool, e.g., a catheter is advanced along a guide wire, thereby inserting the catheter into the bile duct. Thereafter, when replacing the treatment tool, it is necessary to quickly move the guide wire back and forth with the other hand, while grasping the endoscope with one hand. However, this operation is difficult. Therefore, a treatment tool in which an insertion tool configured to insert a guide wire is provided on an outer surface of the catheter has been disclosed (e.g., refer to Patent Document 1).

The insertion tool described in Patent Document 1 is a funnel-shaped lumen having a proximal end opening and a distal end opening. The proximal end opening is an inlet into which the guide wire is inserted, and its inner diameter is formed to be greater than the distal end opening. The distal end opening is connected to the guide wire lumen. With this configuration, it is possible to easily insert the guide wire into the guide wire lumen from the proximal end opening via the distal end opening.

Further, in this treatment tool, an abutting portion (key) is provided at the proximal end side of the guide wire lumen to prevent the guide wire from retracting more than necessary.

### [Citation List]

### [Patent Literature]

[Patent Document 1]
Published Japanese Translation No. 2002-543938 of the PCT International Publication

### [Summary of Invention]

### [Technical Problem]

In the treatment tool disclosed in Patent Document 1, when replacing the treatment tool, the guide wire is retracted (back loaded) to the proximal side from the distal end side of the treatment tool. At this time, because the guide wire collides with the abutting portion, there is a problem that the guide wire is not smoothly exposed from the distal end opening of the proximal side.

The present invention was made in view of such problems, and an object thereof is to provide a treatment tool in which the guide wire can be made to smoothly protrude outward when retracting the guide wire.

### [Solution to Problem]

According to a first aspect of the present invention, a treatment tool includes: a sheath that has a first opening portion which has a longitudinal axis, is formed with a lumen through which a guide wire is capable of being inserted along the longitudinal axis, and is open to communicate on a distal end side of the lumen, and a second opening portion that is opened to an outer circumferential surface to communicate on a proximal end side of the lumen; a proximal end holding portion that holds a proximal end region in the proximal end portion of the sheath; and a distal end holding portion that holds a distal end region of the proximal end portion of the sheath by bending, and is provided by being deflected to the proximal end holding portion, while being continuous with the distal end of the proximal end holding portion at an angle in which the second opening portion is located on the extension of the central axis of the lumen in the distal end region.

According to a second aspect of the present invention, in the treatment tool according to the first aspect, the sheath may have a liquid feed lumen that is capable of feeding fluid, and a knife wire lumen through which a knife wire capable of incising a tissue is inserted, wherein the liquid feed lumen and the knife wire lumen may be disposed at an inward side of the bent portion of the sheath.

According to a third aspect of the present invention, in the treatment tool according to the first aspect, a slit portion may be formed on the outer circumferential surface of the sheath such that the lumen and the outside of the sheath communicate with each other from the second opening portion to the distal end side of the sheath along the direction of the longitudinal axis, and the opening width of the slit portion in the circumferential direction of the sheath is smaller than the inner diameter of the lumen.

According to a fourth aspect of the present invention, in the treatment tool according to the first aspect, an opening portion capable of engaging with the sheath may be formed in the holding portion, a dimension of the opening portion in a direction orthogonal to the longitudinal axis of the holding portion may be smaller than the outer diameter of the sheath, and the opening portion may hold the sheath to change an angle formed between the longitudinal axis of the holding portion and the sheath.

### [Advantageous Effects of Invention]

According to each aspect of the above-mentioned treatment tool, when retracing the guide wire, the guide wire can smoothly protrude outward.

### [Brief Description of Drawings]

Fig. 1 is an overall view showing a treatment tool of a first embodiment of the present invention.
Fig. 2 is a diagram showing a sheath used in the treatment tool of Fig. 1.
Fig. 3 is a cross-sectional view taken along a line III-III shown in Fig. 2.
Fig. 4 is a cross-sectional view taken along a line IV-IV shown in Fig. 2.
Fig. 5 is a diagram showing a holding portion of the treatment tool of Fig. 1.
Fig. 6A is a cross-sectional view taken along a line VI-VI shown in Fig. 5.
Fig. 6B is a cross-sectional view taken along a line X-X of Fig. 6A.
Fig. 6C is a diagram showing a modified example of a cylindrical holding portion.
Fig. 7 is a diagram showing the action of the treatment tool according to the first embodiment of the present invention.
Fig. 8 is a diagram showing the action of the treatment tool according to the first embodiment of the present invention.
Fig. 9 is a diagram showing the action of the treatment tool according to the first embodiment of the present invention.
Fig. 10 is a diagram showing the action of the treatment tool according to the first embodiment of the present invention.
Fig. 11 is a diagram showing the action of a conventional treatment tool.
Fig. 12 is a diagram showing a modified example of the holding portion of the first embodiment of the present invention.
Fig. 13 is a diagram showing a modified example of the holding portion of the first embodiment of the present invention.
Fig. 14 is a diagram showing a modified example of the holding portion of the first embodiment of the present invention.
Fig. 15 is a diagram showing a holding portion of a treatment tool of a second embodiment of the present invention.
Fig. 16 is a diagram in which the holding portion of Fig. 15 is viewed from the bottom.
Fig. 17 is a diagram showing the action of the treatment tool according to the second embodiment of the present invention.

### [Description of Embodiments]

### [First Embodiment]

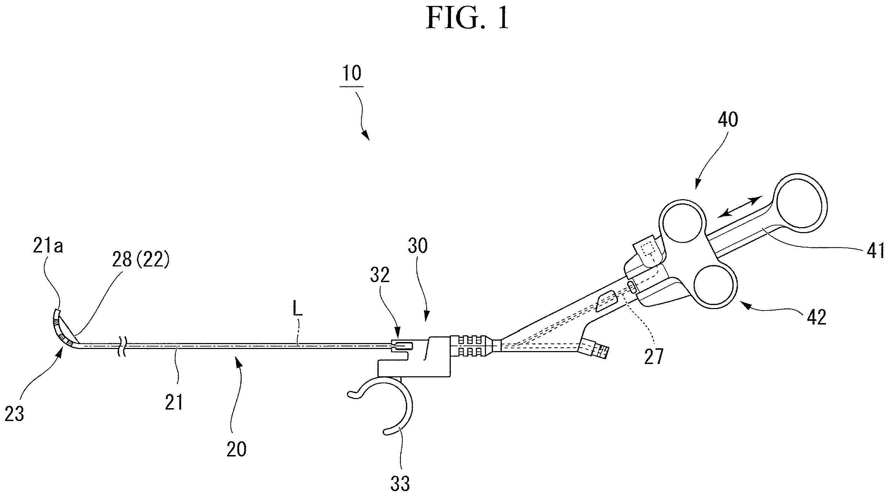

A treatment tool according to a first embodiment of the present invention will be described with reference to Figs. 1 to 14.

As shown in Fig. 1, the treatment tool 10 has an inserting portion 20, a connecting portion 30, and a manipulating portion 40. In the following description, the inserting portion 20 side of the treatment tool 10 will be referred to as a distal end side, and the manipulating portion 40 side will be referred to as a proximal end side.

The inserting portion 20 has a sheath 21 and a knife wire 22, and is inserted into a treatment tool channel of an endoscope apparatus to be described later. The sheath 21 is an elongated member having a longitudinal axis L. Further, the sheath 21 is, for example, made of resin and has flexibility.

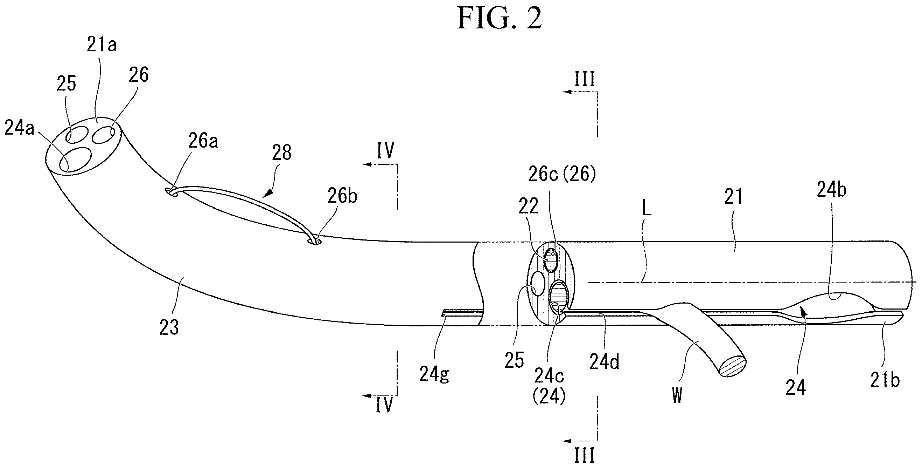

As shown in Fig. 2, the sheath 21 has a pre-curved portion 23 in a region that includes the distal end 21a of the sheath 21. Bending is imparted to the pre-curved portion 23 in a shape that is bent in a predetermined direction, and the pre-curved portion 23 has a restoring force that restores to a preliminarily imparted bent shape.

The distal end portion of the pre-curved portion 23 is inserted into a treatment target. A distal end side communication hole 26a and a proximal end side communication hole 26b (to be described later) are formed on the pre-curved portion 23.

Next, the sheath 21 will be described with reference to Fig. 2. The sheath 21 shown in Fig. 2 shows a state of not being held by a holding portion 32 to be described later.

A first lumen 24, a second lumen 25 and a third lumen 26 are formed inside the sheath 21. The first lumen 24, the second lumen 25 and the third lumen 26 are formed to extend in parallel with one another along a longitudinal axis L of the sheath 21.

The first lumen 24 has a passage portion having an inner diameter which enables the guide wire W to protrude and retract. That is, the first lumen 24 has a lumen that holds the guide wire W inside. Further, the first lumen 24 has an outlet portion (a first opening portion) 24a, an inlet portion (a second opening portion, or an opening portion) 24b, a guide wire housing portion 24c, and a slit portion 24d. The outlet portion 24a of the first lumen 24 is opened to the distal end of the first lumen 24, i.e., the distal end 21a of the sheath 21. The inlet portion 24b is opened to a proximal end of the first lumen 24, i.e., an outer circumferential surface of the proximal end 21b side of the sheath 21.

The slit portion 24d has a shape in which a part of a resin member which constitutes the sheath 21 is cut in the longitudinal direction of the sheath 21, and is formed to the distal end side of the sheath 21 from the inlet portion 24b along the longitudinal axis L direction of the sheath 21. Specifically, the position of the distal end 24g of the slit portion 24d is located on proximal side than the proximal end side communication hole 26b.

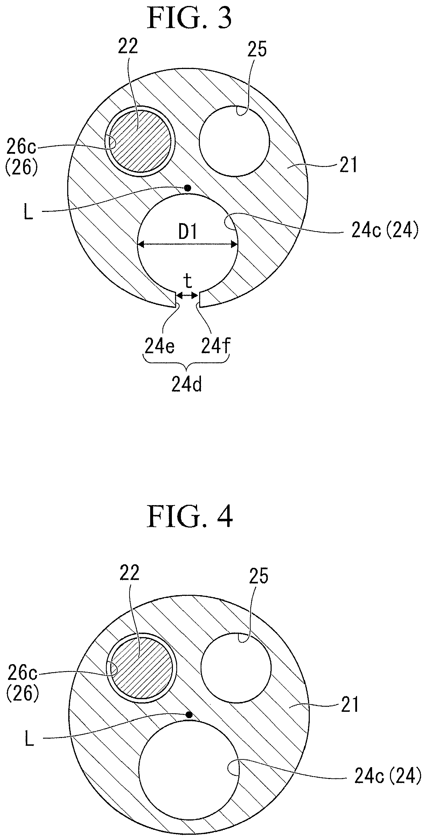

As shown in Fig. 3, the slit portion 24d is formed on the outer circumferential surface of the sheath 21 to make the guide wire housing portion 24c of the first lumen 24 and the outside of the sheath 21 communicate with each other. Furthermore, the slit portion 24d has a pair of a first flap portion 24e and a second flap portion 24f, and the first flap portion 24e and the second flap portion 24f are disposed to be spaced from each other. Specifically, the slit portion 24d is formed so that an opening width t as a gap between the first flap portion 24e and the second flap portion 24f in the circumferential direction of the sheath 21 is smaller than the inner diameter D1 of the first lumen 24. The first flap portion 24e and the second flap portion 24f are elastic portions that cover the guide wire housing portion 24c by a resin member that constitutes the sheath 21.

The first flap portion 24e and the second flap portion 24f are deformable, until a gap with a dimension through which the guide wire W can pass is generated due to the force of a manipulator when detaching the guide wire W from the guide wire housing portion 24c through the slit portion 24d.

The guide wire housing portion 24c has a circular contour except for a boundary with the slit portion in a cross portion orthogonal to the longitudinal axis L of the sheath 21. That is, the periphery of the guide wire housing portion 24c has a substantially C-shaped contour shape in the cross portion orthogonal to the longitudinal axis L of the sheath 21.

The inner diameter of the guide wire housing portion 24c is set to enable the guide wire W to protrude and retract inside the guide wire housing portion 24c, and to have a clearance in a state in which the guide wire W is inserted. That is, the inner diameter of the guide wire housing portion 24c is larger than the diameter by the dimension of the clearance of the guide wire W.

The periphery of the guide wire housing portion 24c has a series of circumferential contours, between the distal end 24g of the slit portion 24d and the distal end 21a of the sheath 21, as shown in Fig. 4, when viewed in the cross portion orthogonal to the longitudinal axis L of the sheath 21. With the present configuration, it is possible to hold the guide wire W so that the guide wire W can protrude from the distal end 21a of the sheath 21.

The second lumen 25 shown in Fig. 2 is provided from the proximal end 21b to the distal end 21a of the sheath 21, and for example, may be used as a liquid feed lumen that feeds a liquid such as a contrast agent. Further, the second lumen 25 may also be utilized as a liquid discharge lumen that removes liquid in the body.

As shown in Fig. 2, the third lumen 26 is a lumen through which the knife wire 22 is inserted. The inner diameter of the third lumen 26 is set such that the knife wire 22 is capable of protruding and retracing and a clearance is available in a state when the knife wire 22 is inserted. That is, the inner diameter of the third lumen 26 is larger than the diameter of the knife wire 22 by the dimension of the clearance.

The third lumen (the knife wire lumen) 26 shown in Fig. 2 includes the distal end side communication hole 26a, the proximal end side communication hole 26b, and the knife wire housing portion 26c that houses the knife wire 22.

As shown in Figs. 1 and 2, the knife wire 22 is provided with a power transmission portion 27 connected to the manipulating portion 40, and an incision portion 28 that is provided on the distal end side of the power transmission portion 27 and is disposed outside the second lumen 25 by the distal end side communication hole 26a and the proximal end side through hole 26b.

As shown in Fig. 1, the manipulating portion 40 includes a rod-like portion 41, and a slider portion 42 connected to the rod-like portion 41. The proximal end of the power transmission portion 27 is connected to the slider portion 42, and by causing the slider 42 to slide with respect to the rod-like portion 41, the incision portion 28 is manipulated.

The connecting portion 30 is provided between the proximal end of the inserting portion 20 and the distal end of the manipulating portion 40.

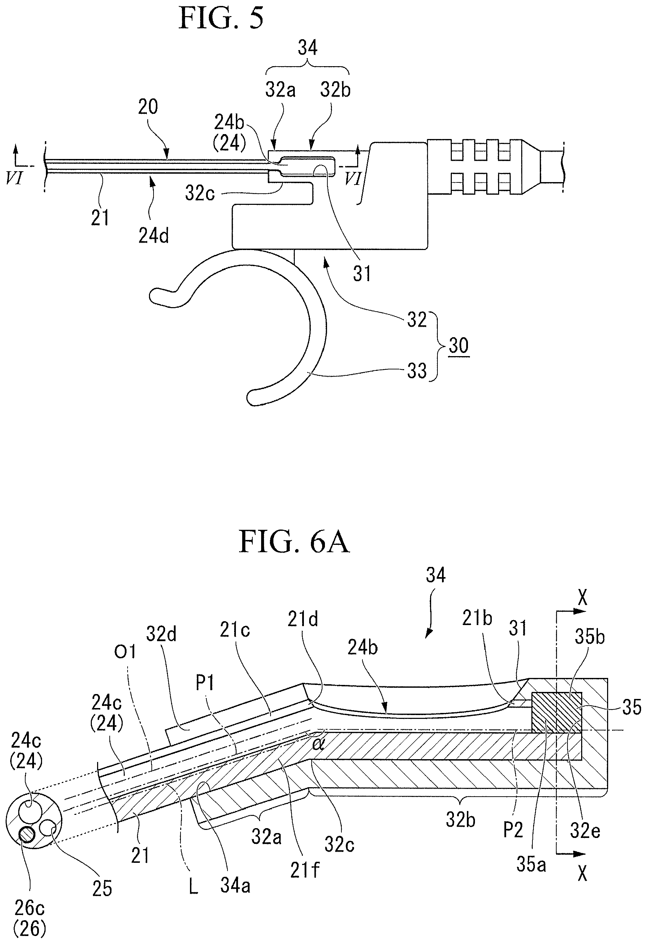

As shown in Fig. 5, the connecting portion 30 is provided with a holding portion 32 and a hook portion 33.

As shown in Fig. 6A, the holding portion 32 has a cylindrical portion 34 formed in a substantially cylindrical shape. Further, the holding portion 32 has a port opening portion 31 formed to communicate with the inner circumferential surface and the outer circumferential surface of the cylindrical portion 34. Further, a groove 32d extends from the distal end of the port opening portion 31 to the most distal end of the holding portion 32. The groove 32d communicates with the distal end opening 34a of the cylindrical portion 34.

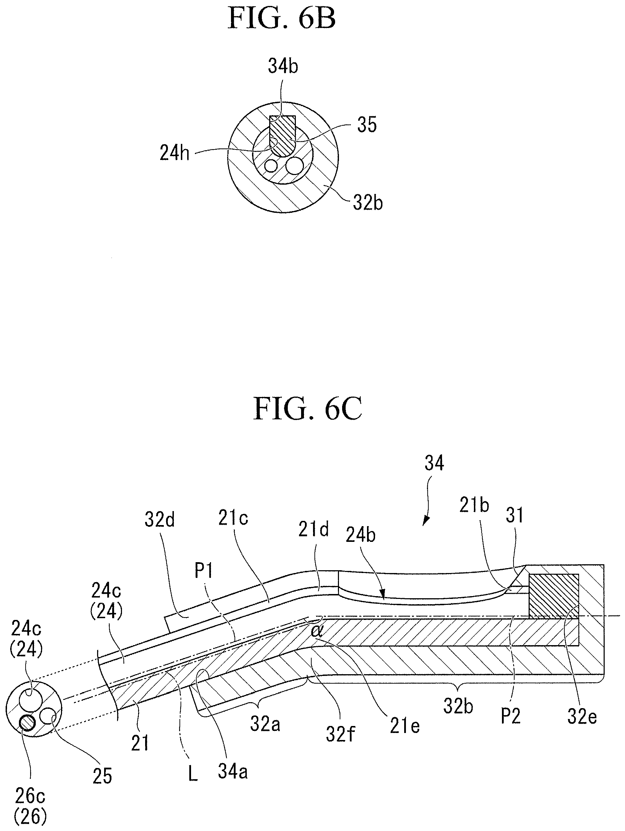

The proximal end portion of the sheath 21 is inserted into the cylindrical portion 34 through the distal end opening 34a. The proximal end portion of the sheath 21 is inserted and fixed into the cylindrical portion 34 of the holding portion 32, such that the opening direction of the port opening portion 31 matches the opening direction of the inlet portion 24b of the port opening portion 31 (the phase of the port opening portion 31 matches the phase of the inlet portion 24b in the longitudinal axis L). Specifically, for example, as shown in Fig. 6A, at the proximal end portion of the cylindrical portion 34, the sheath 21 may be locked to the cylindrical portion 34 such that the sheath 21 does not rotate with respect to the cylindrical portion 34, while the opening direction of the port opening portion 31 and the opening direction of the inlet portion 24b match each other. As shown in Fig. 6B that is a cross-sectional view taken along the line X-X of Fig. 6A, the locking member 35 is locked to both of a locking groove 34b of the cylindrical portion 34 and a locked portion 24h of the first lumen 24. In a direction orthogonal to the longitudinal axis L, one end 35a of the locking member 35 enters the locked portion 24h of the first lumen 24, and the other end 35b of the locking member 35 enters the locking groove 34b of the cylindrical portion 34. Thus, the sheath 21 is locked to the cylindrical portion 34.

Further, in a state in which the opening of the port opening portion 31 and the opening of the inlet portion 24b at least partially overlap each other, the proximal end 21b at the proximal end portion of the sheath 21 is positioned by coming into contact with the end surface 32e. Thus, the opening direction of the port opening portion 31 matches the opening direction of the inlet portion 24b, and the opening of the port opening portion 31 and the opening of the inlet portion 24b overlap each other. Thus, the guide wire W can be inserted into the inlet portion 24b from the port opening portion 31.

Further, in the present embodiment, although the sheath 21 is locked to the cylindrical portion 34 using the locking member 35 so that the relative positional relationship between the sheath 21 and the cylindrical portion 34 is not deviated from a predetermined position, the configuration for locking the sheath 21 to the cylindrical portion 34 is not limited thereto, and the sheath 21 and the cylindrical portion 34 may be locked to prevent the position from deviating.

As shown in Fig. 5, the tubular portion 34 is provided with a distal end holding portion 32a, and a proximal end holding portion 32b.

As shown in Fig. 6A, the distal end holding portion 32a is deflected (inclined) to the proximal end holding portion 32b. Thus, in a state in which the proximal end portion of the sheath 21 is held on the cylindrical portion 34, the opening of the inlet portion 24b overlaps and communicates with the opening of the port opening portion 31, on the extension line of the center axis 01 of the guide wire housing portion 24c of the held portion (distal end region of the proximal end portion of the sheath) 21f of the sheath 21 which is held by the distal end holding portion 32a. Further, the proximal end holding portion 32b holds the proximal end region of the proximal end portion of the sheath. In other words, the longitudinal axis (the longitudinal axis of the sheath 21) P1 of the distal end holding portion 32a intersects with the longitudinal axis (longitudinal axis of the sheath 21) P2 of the proximal end holding portion 32b to form an obtuse angle. That is, the distal end holding portion 32a is inclined with respect to the proximal end holding portion 32b so that the angle α formed between the longitudinal axis P1 and the longitudinal axis P2 is an obtuse angle.

Further, as the configuration in which the distal end holding portion 32a is deflected to the proximal end holding portion 32b, as shown in Fig. 6A, for example, a bent portion (a boundary portion) 32c in which a joint between the proximal end holding portion 32b and the distal end holding portion 32a is bent may be provided. At this time, the port opening portion 31 is provided on proximal side than the sheath 21 with respect to the bent portion 32c. Further, preferably, in a state in which the locking member 35 is locked to both of the holding portion 32 and the locked portion 24h of the first lumen 24, the second lumen 25 and the third lumen 26 are located on the bent portion 32c side.

As shown in Fig. 6C, the cylindrical portion 34 may have a bent portion 32f in which the joint between the distal end holding portion 32a and the proximal end holding portion 32b is gently bent. At this time, the port opening portion 31 may be provided on the bent portion 32f, and may be provided on proximal side than the sheath 21 with respect to the proximal end of the bent portion 32f. Also, preferably, in a state in which the locking member 35 is locked to both of the holding portion 32 and the locked portion 24h of the first lumen 24, the second lumen 25 and the third lumen 26 are located on the side of the inward side 21e of the bent portion 21c of the sheath 21.

In this way, in the state in which the proximal end portion of the sheath 21 is held on the cylindrical portion 34, the bent portion 21c in which the sheath 21 is bent is formed inside the joint between the proximal end holding portion 32b and the distal end holding portion 32a. The distal end holding portion 32a holds the sheath 21 of the distal side than the bent portion 21c (the distal end region of the proximal end of the sheath, the held portion 21f) in which the sheath 21 is bent, and the proximal end holding portion 32b holds the sheath 21 of the proximal side than the bent portion 21c (the proximal end region of the proximal end portion of the sheath). Further, the distal end holding portion 32a is provided to be continuous with the proximal end holding portion 32b.

Although the length of the distal end holding portion 32a in the longitudinal axis direction P1 is not particularly limited, there is a need for enough length to sufficiently deflect the guide wire housing portion 24c in the direction of the extension axis (P1) of the distal end holding portion 32a.

As shown in Fig. 6A, the length of the inlet portion 24b of the proximal end holding portion 32b in the longitudinal axis P2 is preferably greater than the inner diameter of the guide wire housing portion 24c in the first lumen 24. For example, the inlet portion 24b may have a long hole shape along the longitudinal axis L of the sheath 21. The position of the distal end of the inlet portion 24b may be consistent with the bent portion 32c between the distal end holding portion 32a and the proximal end holding portion 32b. Furthermore, the shape of the inlet portion 24b may be an elliptical or circular shape, without being limited to a rectangular shape.

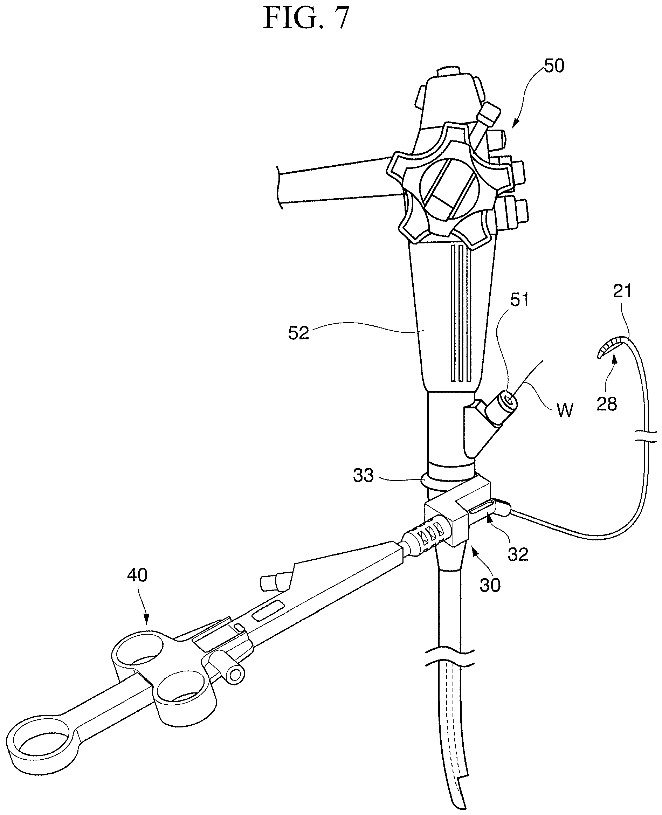

Further, as shown in Figs. 5 and 7, the connecting portion 30 of the treatment tool 10 may have a hook portion 33 that can be locked to a grasped portion 52 of the endoscope apparatus 50. The hook portion 33 is a locking portion that can be locked to the grasped portion 52 of the endoscope apparatus 50, and has a C shape having an inner circumferential surface that forms a through-hole penetrating substantially parallel to the opening direction of the port opening portion 31. The hook portion 33 is an elastic member formed into a C shape such that its inner circumferential surface surrounds a part of the outer circumferential surface of the grasped portion 52 provided in the endoscope apparatus 50. The inner circumferential surface of the hook portion 33 is pressed against the outer surface of the grasped portion 52 of the endoscope apparatus 50, by the restoring force by which the hook portion 33 itself is restored to the C-shape. Therefore, it is possible to attach the manipulating portion 40 of the treatment tool 10 to the grasped portion 52 of the endoscope apparatus 50 via the hook portion 33. In the state of attaching the manipulating portion 40 of the treatment tool 10 to the grasped portion 52 of the endoscope apparatus, in a direction substantially parallel to the longitudinal axis of the grasped portion 52, the inlet portion 24b and the port opening portion 31 communicate with each other. Furthermore, the opening direction of the port opening portion 31 is opened to the manipulating portion (the angle knob) side of the endoscope apparatus.

Next, the operation of the treatment tool 10 according to the present embodiment will be described.

First, the guide wire W is placed in the bile duct from the duodenal papilla using known methods. At this time, as shown in Fig. 7, the distal end of the guide wire W is placed in the bile duct, and the proximal end of the guide wire W is exposed to the outside from the treatment tool channel 51 of the endoscope apparatus 50.

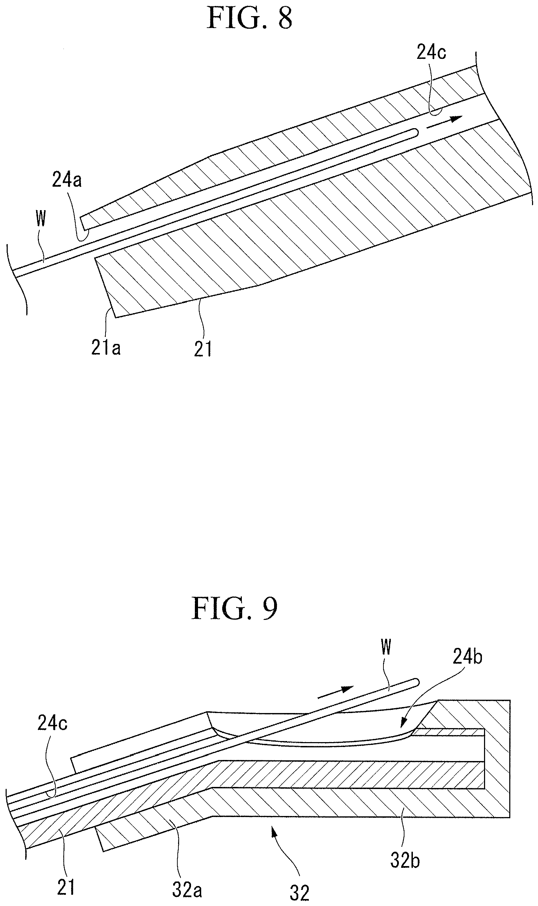

Next, in a state of grasping the hook portion 33 on the grasped portion 52 of the endoscope apparatus 50, or in a state in which the operator grasps the manipulating portion 40, as shown in Fig. 8, the guide wire W exposed to the outside from the treatment tool channel 51 is inserted from the outlet portion 24a provided at the distal end 21a of the sheath 21 of the treatment tool 10. The operator retracts (back loads) the guide wire W along the guide wire housing portion 24c toward the proximal end 21b of the sheath 21.

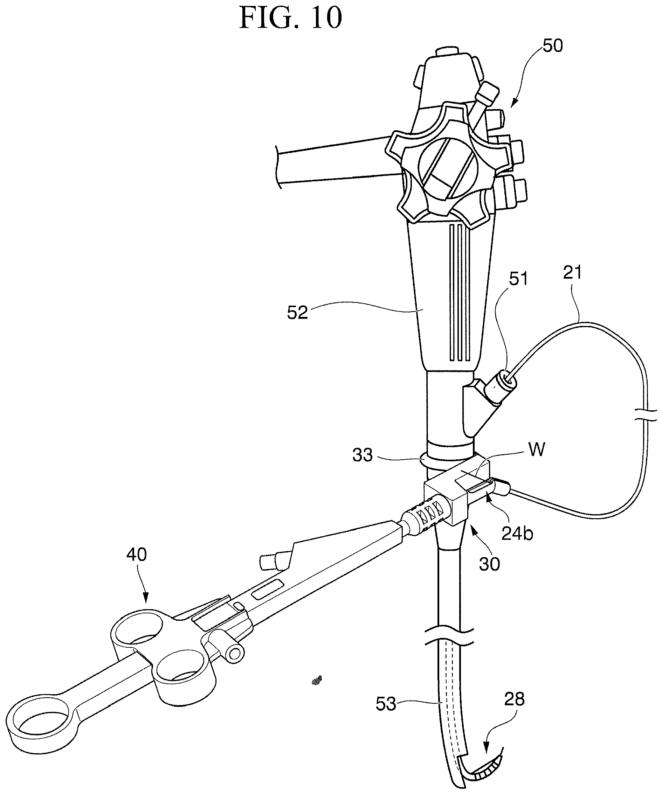

As shown in Fig. 9, although the guide wire W proceeds toward the proximal end holding portion 32b along the sheath 21 held in the distal end holding portion 32a, the guide wire W suitably protrudes outward from the inlet portion 24b due to its rigidity, without proceeding along the sheath 21 held in the proximal end holding portion 32b. Thus, as shown in Fig. 10, the sheath 21 of the treatment tool 10 is inserted into the treatment tool channel 51 of the endoscope apparatus 50, and the incision portion 28 protrudes from the distal end of the inserting portion 53 of the endoscope apparatus 50.

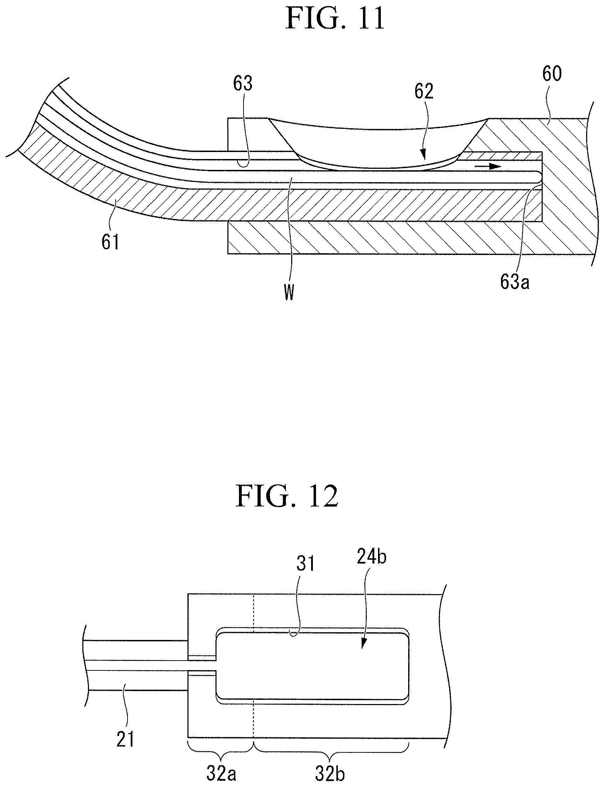

According to the treatment tool 10 of the present embodiment, on the extension straight line of the central axis of the guide wire housing portion 24c in the held portion 21f of the sheath 21 which is held by the distal end holding portion 32a, the distal end holding portion 32a is deflected to the proximal end holding portion 32b so that the opening of the inlet portion 24b and the opening of the port opening portion 31 overlap and communicate with each other. Thus, when back loading the guide wire W, the guide wire W can easily protrude from the inlet portion 24b. That is, as shown in Fig. 11, if the holding portion 60 has a straight line shape that does not have the distal end holding portion 32a and the proximal end holding portion 32b, particularly when the sheath is bent so that the sheath 61 is bent to the inlet portion 62 side, i.e., the inlet portion 62 is located on the inside of the bent shape, the back-loaded guide wire W proceeds toward the end surface 63a without protruding from the inlet portion 62, and it is difficult for it to be exposed from the inlet portion 62.

Therefore, according to the treatment tool 10 of the present embodiment, because the distal end holding portion 32a is inclined (deflected) to the proximal end holding portion 32b, it is possible to prevent the sheath 21 from being bent to the inlet portion 24b side. In addition, even if the sheath 21 applies a load to the inlet portion 24b side, because the longitudinal axis P1 of the distal end holding portion 32a intersects with the longitudinal axis P2 of the proximal end holding portion 32b to form an obtuse angle due to the holding portion 32, the guide wire W can smoothly protrude from the inlet portion 24b.

Further, the first lumen 24 has a slit portion 24d. The slit portion 24d communicates with the port opening portion 31 via the groove 32d extending from the distal end of the port opening portion 31 to the most distal end of the holding portion 32. Therefore, by introducing the guide wire W protruding from the inlet portion 24b into the slit portion 24d, and further, by moving the guide wire W to the distal end side of the slit portion 24d, it is possible to peel off the guide wire W from the sheath 21. The user can grasp and manipulate the guide wire W peeled off from the sheath 21 at an arbitrary position in the longitudinal direction of the sheath 21.

Because the second lumen 25 and the third lumen 26 are disposed on the inside 21e of the bent portion 21c, the second lumen 25 and the third lumen 26 do not interfere with the protrusion of the guide wire W from the inlet portion 24b. Thus, the guide wire W can smoothly protrude from the inlet portion 24b.

Further, in the present embodiment, as shown in Fig. 12, the position of the distal end of the inlet portion 24b may be located on the distal end holding portion 32a side.

Further, although the first lumen 24 is configured to have the slit portion 24d, as shown in Fig. 4, the slit portion 24d may not be provided, and the periphery of the guide wire housing portion 24c may have a series of circumferential contours when viewed in the cross-portion orthogonal to the longitudinal axis L of the sheath 21 from the distal end to the proximal end of the sheath 21. That is, a shape which is only opened to the distal end and the proximal end of the sheath 21 may be provided.

### [Modified Example]

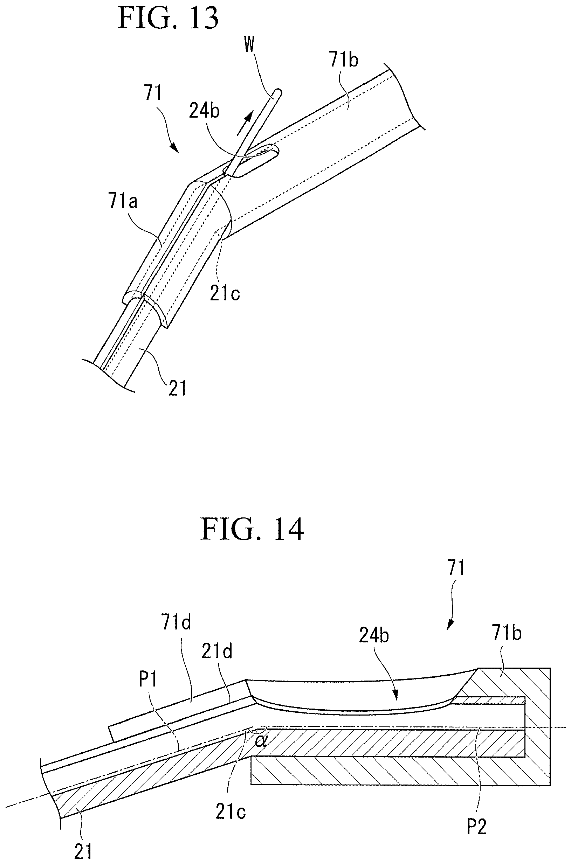

A treatment tool of a modified example of the present invention will be described with reference to Figs. 13 and 14.

Because the configuration of the holding portion of the treatment tool of the present modified example is different from the holding portion of the first embodiment, these differences will be described.

In the treatment tool, as shown in Fig. 13, the holding portion 71 includes a distal end holding portion 71a and a proximal end holding portion 71b. The distal end holding portion 71a and the proximal end holding portion 71b are separately provided, and the proximal end side of the distal end holding portion 71a is connected to the distal end side of the proximal end holding portion 71b.

The distal end holding portion 71a holds the distal end side of the bent portion 21c of the sheath 21, and the proximal end holding portion 71b holds the proximal end side of the bent portion 21c of the sheath 21.

Further, as shown in Fig. 14, when the holding portion 71 is viewed from one direction perpendicular to the longitudinal axis L of the sheath 21, the longitudinal axis (the longitudinal axis of the sheath 21) P1 of the distal end holding portion 71a intersects with the longitudinal axis (the longitudinal axis of the sheath 21) P2 of the proximal end holding portion 71b to form an obtuse angle. That is, the distal end holding portion 71a is inclined with respect to the proximal end holding portion 71b so that the angle α formed between the longitudinal axis P1 and the longitudinal axis P2 is an obtuse angle. The same also applies to the first embodiment.

According to the modified example, the holding unit 71 holds the sheath 21 so that the inlet portion 24b is located on the outward side 21d of the bent shape of the bent portion 21c of the sheath 21. At this time, the distal end holding portion 32a is deflected (inclined) with respect to the proximal end holding portion 32b so that the opening of the inlet portion 24b overlaps and communicates with the opening of the port opening portion 31, on the extension straight line of the central axis of the guide wire housing portion 24c in the held portion 21 f of the sheath 21 which is held by the distal end holding portion 32a. Therefore, when back-loading the guide wire W, the guide wire W can smoothly protrude from the inlet portion 24b.

Also, because the distal end holding portion 71a and the proximal end holding portion 71b are separately provided, it is also possible to connect the distal end holding portion 71a to the proximal end holding portion 71b only when back-loading the guide wire W.

### [Second Embodiment]

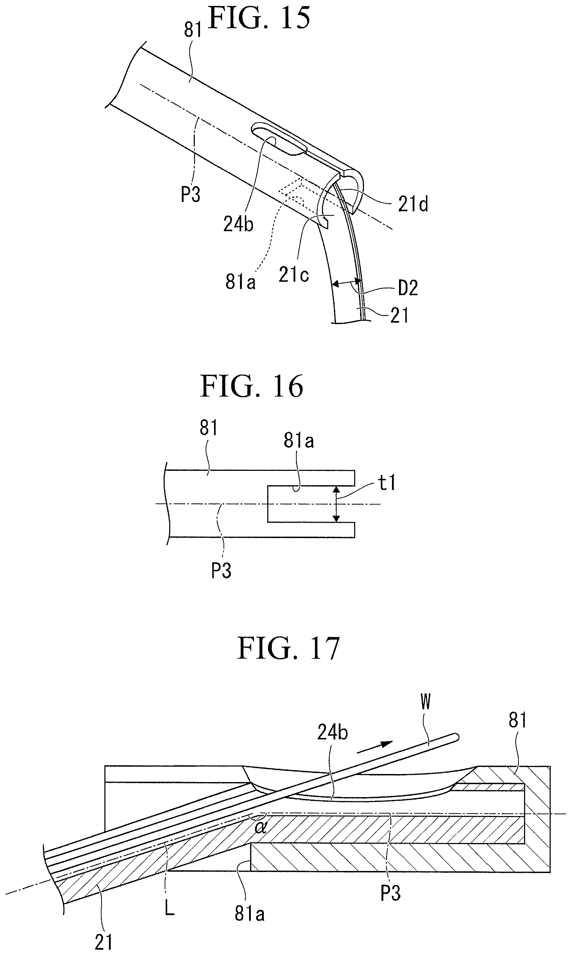

A second embodiment of the present invention will be described with reference to Figs. 15 to 17.

The treatment tool of the embodiment differs from the first embodiment in the configuration of the holding portion.

In the following description, the same constituent elements as those described above are denoted by the same reference numerals, and repeated description will not be provided.

As shown in Fig. 15, an opening portion (notch) 81a capable of engaging with the sheath 21 is formed on the distal end side of the holding portion 81. The opening portion 81a is formed on the side of the holding portion 81 opposite to the inlet portion 24b. As shown in Fig. 16, a dimension (a width) tl of the opening portion 81a in the direction orthogonal to the longitudinal axis P3 of the holding portion 81 is set to be smaller than an outer diameter D2 of the sheath 21 shown in Fig. 15. With the present configuration, the sheath 21 can engage with the opening portion 81a so that the angle α formed between the longitudinal axis P3 of the holding portion 81 and the longitudinal axis L of the sheath 21 of the proximal side than the opening portion 81a forms a desired angle (90 degrees or more and less than 180 degrees). That is, the sheath 21 is held in the opening portion 81 a so that the angle α formed between the longitudinal axis P3 of the holding portion 81 and the longitudinal axis L of the sheath 21 can be adjusted to form the desired angle.

Further, the holding unit 81 holds the sheath 21 so that the inlet portion 24b is located on the outward side 21d of the bent shape of the bent portion 21c of the sheath 21. At this time, the distal end holding portion 32a is deflected (inclined) with respect to the proximal end holding portion 32b so that the opening of the inlet portion 24b overlaps and communicates with the opening of the port opening portion 31, on the extension straight line of the central axis of the guide wire housing portion 24c in the held portion 21f of the sheath 21 which is held by the distal end holding portion 32a.

Next, the operation of the treatment tool according to the present embodiment will be described.

Like the first embodiment, the guide wire W is placed in the bile duct from the duodenal papilla, using known methods.

Next, the sheath 21 is engaged with the opening portion 81a of the holding portion 81 at a desired angle. Further, like the first embodiment, the guide wire W exposed outward from the treatment tool channel 51 of the endoscope apparatus 50 is inserted from the outlet portion 24a provided at the distal end 21a of the sheath 21 of the treatment tool. The operator retracts (back loads) the guide wire W toward the proximal end 21b of the sheath 21.

As shown in Fig. 17, the guide wire W proceeds along the sheath 21 engaged with the opening portion 81a, and protrudes outward from the inlet portion 24b.

According to the treatment tool of the embodiment, like the first embodiment, the distal end holding portion 32a is deflected with respect to the proximal end holding portion 32b so that the opening of the inlet portion 24b overlaps and communicates with the opening of the port opening portion 31, on the extension straight line of the central axis of the guide wire housing portion 24c in the held portion 21 f of the sheath 21 which is held by the distal end holding portion 32a. Therefore, when back-loading the guide wire W, the guide wire W can easily protrude from the inlet portion 24b.

In addition, since the angle α formed between the longitudinal axis P3 of the holding portion 81 and the longitudinal axis L of the sheath 21 is variable, it is also possible to tilt the sheath 21 with respect to the longitudinal axis P3 of the holding portion 81, only when the guide wire W is exposed from the inlet portion 24b, by engaging the sheath 21 with the opening portion 81a.

Furthermore, it is also possible to finely adjust the angle α depending on the characteristics of the guide wire W.

While the preferred embodiments of the present invention have been described, the present invention is not limited to these embodiments. Additions, omissions, substitutions and other modifications of the configurations can be made within the scope that does not depart from the gist of the present invention. The present invention is not limited by the foregoing description, and is only limited by the scope of the appended claims.

For example, while the treatment tool having the incision portion has been described as an example, for example, a treatment tool provided with a balloon catheter may be provided, without being limited thereto. Further, while the treatment tool provided with the first lumen, the second lumen and the third lumen has been described as an example, a lumen through which at least the guide wire can be inserted may be provided.

### [Industrial Applicability]

According to the treatment tool of each embodiment (including the modified example), when retracing the guide wire, the guide wire can smoothly protrude outward.

### [Reference Signs List]

W Guide wire
1 treatment tool
21 sheath
21a distal end of sheath
21b proximal end of sheath
21c bent portion of sheath
21d outward side of bent portion of sheath
21e inward side of bent portion of sheath
24 first lumen
24a outlet portion (first opening portion)
24b inlet portion (second opening portion)
24d slit portion
32, 71, 81 holding portion
32a distal end holding portion
32b proximal end holding portion
71a distal end holding portion
71b proximal end holding portion

## Claims

1. A treatment tool comprising:
a sheath that has a distal end portion and a proximal end portion, is formed with a lumen through which a guide wire is capable of being inserted from the distal end portion to the proximal end portion, and has an opening portion communicating with the lumen on an outer circumferential surface of the proximal end portion;
a proximal end holding portion that holds a proximal end region at the proximal end portion of the sheath; and
a distal end holding portion that bends and holds a distal end region of the proximal end portion of the sheath, and is disposed by being deflected to the proximal end holding portion while being continued to a distal end of the proximal end holding portion at an angle in which the opening portion is located on the extension of the central axis of the lumen in the distal end region.

2. The treatment tool according to claim 1, wherein the sheath has a liquid feed lumen that is capable of feeding fluid, and
a knife wire lumen through which a knife wire capable of incising a tissue is inserted, and
wherein the liquid feed lumen and the knife wire lumen are disposed at an inward side of the bent portion of the sheath.

3. The treatment tool according to claim 1, wherein a slit portion through which the lumen and the exterior of the sheath communicate with each other is formed, an opening width of the slit portion in a circumferential direction of the sheath being smaller than an inner diameter of the lumen, and the slit portion being formed from the opening portion to the distal end portion of the sheath along a longitudinal axis of the sheath.

4. The treatment tool according to claim 1, wherein a notch capable of engaging with the sheath is formed at the proximal end holding portion,
wherein a width of the notch in the direction orthogonal to the longitudinal axis of the proximal end holding portion is smaller than an outer diameter of the sheath, and
wherein the notch holds the sheath such that the angle formed between the longitudinal axis of the proximal end holding portion and the longitudinal axis of the sheath is adjustable.
